(19) Europäisches Patentamt
European Patent Office
Office européen des brevets

(11) Numéro de publication: **0 091 838**
**B1**

(12) # FASCICULE DE BREVET EUROPEEN

(45) Date de publication du fascicule du brevet: **09.04.86**

(51) Int. Cl.⁴: **C 07 D 207/08**, G 02 F 1/35

(21) Numéro de dépôt: **83400155.4**

(22) Date de dépôt: **21.01.83**

(54) **Nouveaux dérivés de la paranitroaniline utilisables en optique non linéaire et en électro-optique et leur procédé de préparation.**

(30) Priorité: **26.01.82 FR 8201166**

(43) Date de publication de la demande:
**19.10.83 Bulletin 83/42**

(45) Mention de la délivrance du brevet:
**09.04.86 Bulletin 86/15**

(84) Etats contractants désignés:
**DE GB NL**

(56) Documents cités:
**JOURNAL OF THE AMERICAN CHEMICAL SOCIETY, vol. 88, no. 18, 20 septembre 1966, pages 4254-4257 W. OELOFSEN et al.: "Adrenocorticotropins. XXXVI. Synthesis of a biologically active prolinol analog of the nonadecapeptide corresponding to the amino-terminal portion of the ACTH molecule"**
**OPTICS COMMUNICATIONS, vol. 15, no. 2, octobre 1975, pages 258-262 J.L. OUDAR et al.: "Second-order polarizabilities of some aromatic molecules"**
**INSTRUMENTAL AND EXPERIMENTAL TECHN., vol. 22, no. 1, 1er février 1979, pages 202-204, Plenum Publishing Corporation V.P. ERMAKOV et al.: "Installation for the measurements of the intensity of the second optical harmonic of laser radiation in a liquid"**

(73) Titulaire: **Nicoud, Jean-François, 32 rue Gometz, F-91140 Bures-sur-Yvette (FR)**
Titulaire: **Zyss, Joseph, 58, rue Desaix, F-75015 Paris (FR)**

(72) Inventeur: **Nicoud, Jean-François, 32 rue Gometz, F-91140 Bures-sur-Yvette (FR)**
Inventeur: **Zyss, Joseph, 58, rue Desaix, F-75015 Paris (FR)**

(74) Mandataire: **Mongrédien, André et al, c/o BREVATOME 25, rue de Ponthieu, F-75008 Paris (FR)**

**EP 0 091 838 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention a pour objet de nouveaux derivés de la paranitroaniline, utilisables en optique non linéaire et en électro-optique, et leur procédé de préparation.

Par le terme optique non linéaire, on entend le domaine de l'optique qui s'étend de la conversion des fréquences optiques (obtention d'un rayonnement optique à partir de deux rayonnements de fréquence différente, la fréquence du rayonnement de conversionetant égale à la somme ou à la différence des fréquences des deux rayonnements) à la modulation électrooptique (modification d'une des caractéristiques d'un rayonnement par application d'un champ électrique à un cristal traverse par ledit rayonnement).

On connaît déjà de nombreux matériaux susceptibles de convenir à la conversion des fréquences optiques ou à la modulation électro-optique. parmi ceux-ci, les cristaux doubleurs de fréquence à base de diphosphate de potassium KDP ou de niobate de lithium sont les plus utilises.

Cependant, ces matériaux minéraux présentent l'inconvénient de manquer d'efficacité, ce qui oblige à les employer sous de fortes épaisseurs. Aussi, depuis quelques années, les recherches ont porté sur la realisation de cristaux de molécules organiques présentant une efficacité améliorée par rapport aux cristaux mineraux de diphosphate de potassium ou de niobate de lithium. Ainsi, on a trouvé que des dérivés d'aniline comme la métanitroaniline, la 2-méthyl-4-nitroaniline, la 2-chloro-4-nitroaniline, la 2-bromo-4-nitroaniline et le 2(2,4-dinitrophényl)-aminopropanoate de methyle pouvaient être utilisés dans ce but. De même, on a découvert les propriétés intéressantes des dérivés de la pyridine-N-oxyde, par exemple de la 3-méthyl-4-nitropyridine-1-oxyde (voir "A molecular engineering approach toward the design of efficient organic crystals for three-wave mixing", J.Zyss in Current Trends in Optics p. 122 (Taylor et Francis, Londres 1981); B.L. Davydov et al., ZhETF Pis. Red. 12(1), 24 (1970); et B.F. Levine, C.G. Bethea, C.D. Thurmond, R.T. Lynchet L. Bernstein, J. Appl. Phys. 50, 2523 (1979).

La présente invention a pour objet de nouveaux composés organiques dérivés de la paranitroaniline qui présentent par rapport aux composés connus une meilleure efficacité en optique non linéaire.

Selon un premier mode de réalisation de l'invention, le dérivé de la paranitroaniline répond à la formule suivante:

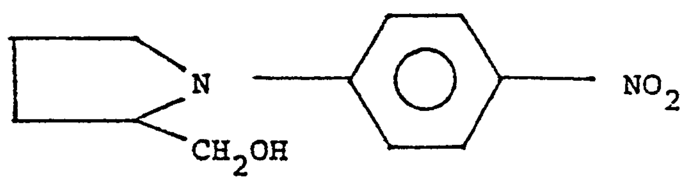

(I)

Selon un second mode de réalisation de l'invention, le dérivé de la paranitroaniline répond à la formule (I) précitée dans laquelle un ou plusieurs des atomes d'hydrogène sont remplacés par des atomes de deuterium.

Le dérivé de la paranitroaniline repondant à la formule (I) peut être preparé en faisant réagir la 2-(hydroxymethyl)pyrrolidine communément appelé "prolinol" de formule II

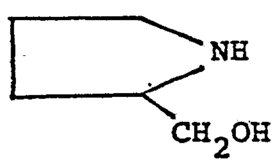

(II)

avec un parahalogénonitrobenzène de formule III

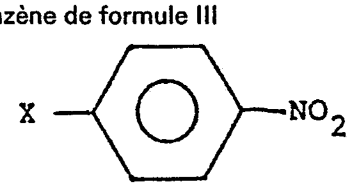

(III)

dans laquelle X représente un atome d'halogène, de preférence le fluor, dans un solvant approprié pour ce type de substitution nucléophile aromatique.

A titre de solvant susceptible d'être utilisée, on pent citer le dimethylsulfoxyde (DMSO) et le diméthylformamide (DMF).

Le mode opératoire adopté pour réaliser cette réaction, est analogue à celui décrit par H.Smith et al., J.Am.Chem. Soc. 101, p. 5186-5193 (1979), et H. Bader et al, J. Org. Chem., 31, p. 2319-2321, (1966).

Les dérivés de la paranitronaline répondant à la formule (I) dans laquelle un ou plusieurs des atomes d'hydrogène sont remplacés par des atomes de deutérium, peuvent être préparés par deutération partielle ou totale du dérivé de la paranitroaniline obtenu par réaction de la 2-(hydroxyméthyl)pyrrolidi-ne de formule (II)

avec un parahalogénonitrobenzène de formule (III).

Cette réaction de deutération peut être effectuée par des méthodes classiques d'échange isotopique, par exemple par échange avec de l'eau lourde $D_2O$, par réaction avec DCl et $AlCl_3$, par traitement prolongé en présence de $D_2SO_4$ ou par traitement dans l'ammoniac liquide deuteré en présence d'amidure de sodium comme catalyseur. Ces méthodes sont décrites dans "Isotopic exchange and the replacement of hydrogen in organic compounds" (traduit du russe) A.I. SHATENSHTEIN Consultant Bureau N.Y. (1962) - Appendix p. 295-308.

On peut aussi obtenir les dérivés deuterés du composé de formule (I) par synthese en faisant réagir de la 2-(hydroxyméthyl)pyrrolidine deutérée partiellement ou totalement avec un parahalogénonitrobenzène de formule (III) éventuellement deuteré.

Les composés deuterés de formule (II) ou de formule (III) peuvent être obtenus par les procédés décrits ci-dessus.

Le composé de formule (II) utilise comme produit de départ pour la synthese des dérivés de paranitroaniline de l'invention peut être obtenu à partir de la L-proline ou S-proline qui est un acide aminé naturel appartenant à la serie L, pour lequel le carbone asymétrique est de configuration absolue S, suivant la nomenclature officielle de la stéréochimie organique (voir publication IUPAC, J. Org. Chem. 35, 2849-2867 (1970) Appendix 2). Ainsi, on obtient directement la L-N(4-nitrophényl)-2(hydroxymethyl)pyrro-lidine de formule (I).

Toutefois, on peut utiliser de la même façon l'autre énantiomere du compose de formule (II) issu de l'énantiomere non naturel de la proline, appartenant à la série D, pour lequel le carbone asymétrique est de configuration absolue R, ce qui permet d'obtenir directement le composé de l'invention de formule (I) de configuration absolue R, qui présente led mêmes propriétés que celles du dérivé S, en optique non linéaire.

Lorsque le composé de formule (II) est preparé à partir de la proline non naturelle, celui-ci est obtenu sous la forme du racémique, c'est-à-dire du mélange équimolaire des deux énantiomeres.

Dans ce cas, on peut dédoubler le racemique par des procédés classiques afin d'utiliser l'un des composés optiquement purs de formule (II) pour preparer le composé de formule (I) de l'invention. Toutefois, on peut aussi utiliser le racémique pour préparer le composé de formule (I) qui sera obtenu dans ce cas sous la forme du racémique, et dédoubler ensuite le racémique pour obtenir les composes optiquement purs.

Les dérivés de la paranitroaniline de l'invention, deuterés ou non, présentent une structure leur conferant une non linearité optique très élevée. En effet, ces molécules possedent un système d'électrons $\pi$ délocalisés dissymétriques de grande mobilite; la présence d'un groupement accepteur d'électrons ($NO_2$) et d'un groupement donneur d'électrons ( $>\tilde{N}$- ) liés aucycle benzénique permet d'obtenir une forte conjugaison et un transfert de charge entre le substituant donneur d'électrons et le substituant accepteur d'électrons.

Par ailleurs, le fait que l'atome d'azote du groupement donneur d'electrons soit disubstitue, permet de rendre la molecule moins sensible aux réactions de degradation, qui sont géneralement liées à la presence de liaisons N-H.

Grâce à la présence d'un carbone asymétrique S (ou R) dans le cycle pyrrolidine, les composés de l'invention présentent une structure cristalline non centrosymétrique, qui permet d'obtenir la non linéarité du deuxième ordre.

De plus, la structure cristalline du composé de l'invention, présente des caractéristiques optimales pour obtenir l'efficacité non linéaire. En effet, le groupe d'espace du composé cristallin est $P2_1$, et l'angle de l'axe de transfert de charge qui relie l'azote du groupement donneur d'électrons à l'azote du groupement accepteur d'électrons, avec l'axe de symétrie est de 58°, c'est-à-dire très voisin de l'angle optimum théorique de 54°, ce qui permet d'obtenir un gain sur l'efficacité non linéaire par rapport à une molécule identique dont les groupements donneur et accepteur d'électrons seraient positionnes differemment.

De plus les composés de l'invention ont un édifice cristallin consolidé grâce à la présence dans la molécule d'une fonction alcool, c'est-à-dire d'un groupement polaire. Dans le réseau cristallin, ces groupements polaires peuvent établir des liaisons hydrogène intermoléculaires qui améliorent la cohesion.

En conséquence, lorsque les composés de l'invention sont sous forme cristalline, ceux-ci possedent une bonne tenue mecanique et ils resistent bien à differents traitements mecaniques tels que la taille et le polissage.

Enfin, étant donné que l'on peut préparer les composés de l'invention à partir de la S(+)2-(hydroxyméthyl)pyrrolidine qui est issue d'un acide amine naturel et se présente de ce fait sous une forme optiquement pure, les composés de l'invention peuvent être obtenus directement sous la forme de cristaux optiquement purs, ce qui permet d'éviter les opérations intermédiaires de dédoublement chimique.

Lorsque le composé de l'invention de formule (I) se présente sous la forme du racémique, c'est-à- dire du mélange équimolaire des deux enantiomères, celui-ci peut cristalliser sous deux formes:

a) - le racémate qui correspond à une juxtaposition de molécules droites et gauches dans la maille, ce qui conduit, soit à un système centrosymétrique, soit à un système non centrosymétrique, mais au moins à miroir, qui est différent de celui du cristal optiquement pur ayant la structure $P2_1$;

b) - le conglomérat qui correspond à une juxtaposition de microcristaux droits et gauches. Dans ce dernier cas, on peut obtenir un dédoublement spontane conduisant aux cristaux optiquement purs droits ou gauches à structure $P2_1$ (voir A. Collet, M.J. Brienne et J. Jacques, Chemical Reviews, 80 (3), 215, (1980)).

Les composés de l'invention à l'état optiquement purs, peuvent être utilisés en optique non linéaire sous différentes formes, par exemple sous la forme de poudres, sous la forme d'inclusions de molécules dans un reseau hôte (polymère, clathrate, solution solide), sous la forme de couches minces déposées sur un substrat, sous la forme de monocristal, sous la forme de solutions etc... Les composés de l'invention à l'état optiquement pur sont utilisables dans des dispositifs optiques ou opto-électroniques mettant en jeu les effets physiques

suivants

- les effets non linéaires du premier ordre, c'est-àdire toute interaction à 3 photons dans la plage de transparence du matériau, par exemple génération du second harmonique, somme, différence et transposition de fréquences, gain et oscillations paramétriques, et
- les effets électro-optiques.

Pour ces différentes applications, les composés de l'invention sont tout d'abord purifiés, puis mis sous la forme voulue par exemple sous la forme de monocristaux par des procédés classiques, par exemple par évaporation de solvant ou par une méthode de croissance en solution par refroidissement contrôlé. Lorsqu'on veut les utiliser sous la forme de couches minces, on peut préparer de telles couches par la méthode de Langmuir-Blodgett ou encore par épitaxie.

D'autres caractéristiques et avantages de l'invention apparaîtront mieux à la lecture de l'exemple suivant donné bien entendu à titre illustratif et non limitatif.

Cet exemple illustre la préparation de S(-)N-(4-nitrophényl)-2-(hydroxyméthyl)pyrrolidine, c'est-à-dire du composé de l'invention répondant à la formule (I).

On prépare une première solution en mélangeant 25 g (0,25 mole) de 5(+)2-(hydroxyméthyl)pyrrolidine ou S(+)-prolinol avec 25 ml de diméthyl-sulfoxyde (DMSO) fraichement distille et anhydre.

On prépare une seconde solution en dissolvant par ailleurs 33 g (0,235 mole) de 4-fluoronitro-benzène dans 25 ml de DMSO, puis on verse goutte à goutte cette seconde solution dans la premiàre solution en agitant et en refroidissant au bain d'eau froide, apràs avoir ajoute un equivalent environ (17 g) de carbonate de potassium ($K_2CO_3$). On chauffe ensuite le mélange reactionnel à 60° C tout en maintenant l'agitation. Il se produit alors un dégagement gazeux qui provoque une mousse abondante, et on laisse le tout sous agitation à 60°C pendant 15 heures. Après refroidissement on verse le mélange réactionnel dans 800 ml d'eau glacée sous une agitation énergique que l'on maintient pendant une heure. Il se forme un précipité jaune que l'on filtre sur verre fritté. On reprend le précipité jaune ainsi séparé dans 500 ml de chlorure de methylène ($CH_2Cl_2$) jusqu'à dissolution complète et en chauffant si cela est nécessaire. On laisse decanter la phase aqeuese surnageante et on sèche la phase organique avec du sulfate de magnesium anhydre ($MgSO_4$). Après séchage, on évapore le solvant et on obtient 48 g d'un solide jaune orange, ce qui correspond à un rendement de 92 %.

On purifie ensuite le produit par recristallisation. Dans ce but, on dissout le produit jaune orange dans 200 ml de chloroforme à chaud et au reflux, jusqu'à l'obtention d'une solution homogène et claire, puis on ajoute petit à petit jusqu'à 100 ml de cyclohexane très chaud, jusqu'à la limite de précipitation. On porte alors au reflux jusqu'à l'obtention d'une solution claire et on laisse recristalliser. On recueille ainsi 46 g de produit jaune cristallise, présentant un point de fusion égal à 118°C et ayant une bonne purete. Le rendement global en produit recristallise est de 87 %. Le pouvoir rotatoire mesure en solution dans l'éthanol absolu à une concentration c égale à 1, est:

$$[\alpha]_{589}^{18} = -\ 92,1^0$$

$$[\alpha]_{578}^{18} = -\ 97,0^0$$

$$[\alpha]_{546}^{18} = -\ 113,2^0$$

Le groupe d'espace de la phase cristalline du composé obtenu est $P2_1$ et le composé presente une transparence cristalline dans le domaine de 0,5 micron à 2 microns. Le test standard de génération du second harmonique effectué sur la poudre de ce composé selon la méthode décrite dans: S.K.Kurtz, T.T.Perry, J. Appl. Phys. 39,8, p. 3798 (1968), conduit à un rendement sensiblement égal à $10^4$ fois celui du quartz. On a réalisé ce test en utilisant comme source la raie à 1,06 micron d'un laser YAG: $Nd^{3+}$ et en plaçant l'echantillon au foyer d'un reflecteur parabolique.

Le critère de mérite ($d^2/n^3$) est donc d'environ deux ordres de grandeur supérieur à celui du niobate de lithium.

Ainsi, ce composé présente une non linéarité beaucoup plus forte que celle du 2-(2,4-dinitrophényl)-aminopropanoate de méthyle qui est l'un des meilleurs composés connus actuellement.

**Revendications**

1. Dérivé de la paranitroaniline répondant à la formule suivante:

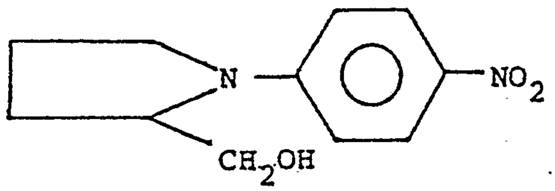

(I)

2. Dérivé de la paranitroaniline repondant à la formule suivante:

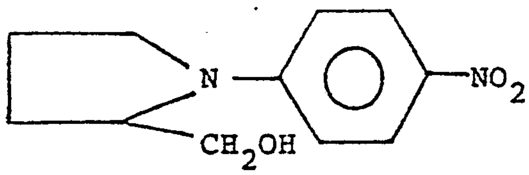

(I)

dans laquelle un ou plusieurs des atomes d'hydrogène sont remplacés par des atomes de deuterium.

3. Procédé de preparation du dérivé de la paranitroaniline selon la revendication 1, caracterise en ce qu'il consiste à faire réagir la 2-(hydroxy-méthyl)pyrrolidine de formule II

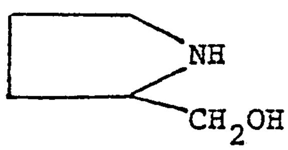

(II)

avec un parahalogénonitrobenzène de formule III

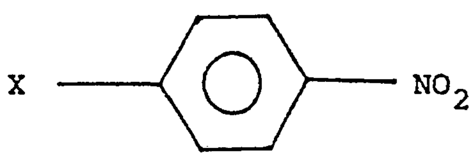

(III)

dans laquelle X représente un atome d'halogène.

4. Procédé selon la revendication 3, caractérisé en ce que X représente le fluor.

5. Procédé de préparation du dérivé de la paranitroaniline selon la revendication 2, caractérisé en ce qu'il consiste à faire réagir la 2-(hydroxy-méthyl)pyrrolidine de formule II

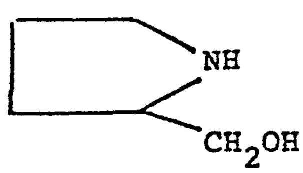

(II)

avec un parahalogénon-itrobenzène de formule III

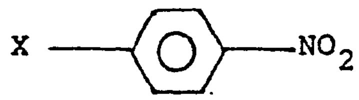

(III)

dans laquelle X représente un atome d'halogène et à deutérer le dérivé de la paranitroaniline ainsi obtenu.

6. Procédé selon l'une quelconque des revendications 3 à 5, caractérisé en ce que l'on utilise l'un ou l'autre des deux énantiomères S(+) ou R (−) de la 2-(hydroxyméthyl)pyrrolidine.

7. Utilisation des dérivés de la paranitroaniline optiquement purs selon l'une quelconque des revendications 1 et 2 dans des dispositifs optiques ou opto-électroniques.

**Patentansprüche**

1. Paranitroanilin-Derivat der Formel:

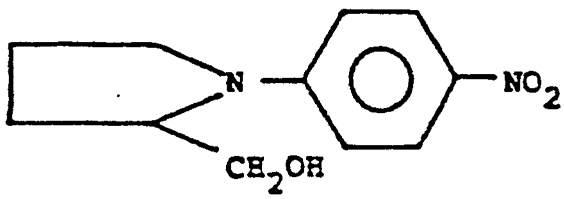

(I)

2. Paranitroanilin-Derivat der Formel:

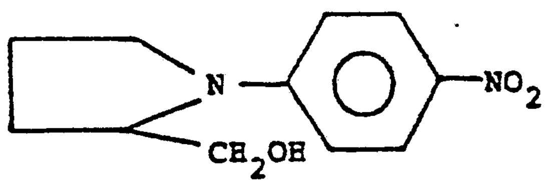

(I)

worin eines oder mehrere der Wasserstoffatome durch Deuteriumatome ersetzt ist bzw. sind.

3. Verfahren zur Herstellung des Paranitroanilin-Derivats nach Anspruch 1, dadurch gekennzeichnet, daß man 2-(Hydroxymethyl)pyrrolidin der Formel II

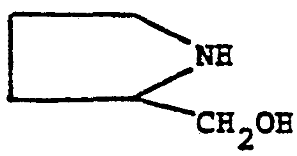

(II)

mit einem Parahalogennitrobenzol der Formel III reagieren läßt

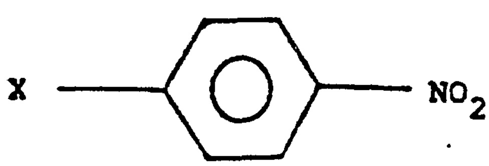

(III)

worin X ein Halogenatom darstellt.

4. Verfahren nach Anspruch 3, dadurch gekennzeichnet, daß X Fluor darstellt.

5. Verfahren zur Herstellung des Paranitroanilin-Derivats nach Anspruch 2, dadurch gekennzeichnet, daß man

2-(Hydroxymethyl)pyrrolidin der Formel II

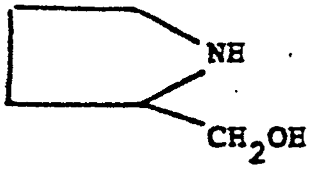

(II)

mit einem Parahalogennitrobenzol der Formel III reagieren läßt

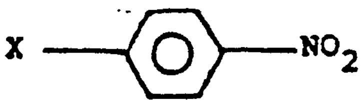

(III)

worin X ein Halogenatom darstellt, und das dabei erhaltene Paranitroanilin-Derivat deuteriert.

6. Verfahren nach einem der Ansprüche 3 bis 5, dadurch gekennzeichnet, daß man das eine oder das andere der beiden S(+)- oder R(−)-Enantiomeren von 2-(Hydroxymethyl)-pyrrolidin verwendet.

7. Verwendung der optischen reinen Paranitroanilin-Derivate nach einem der Ansprüche 1 und 2 in optischen oder elektro-optischen Einrichtungen.

**Claims:**

1. Paranitroaniline derivative having the following formula:

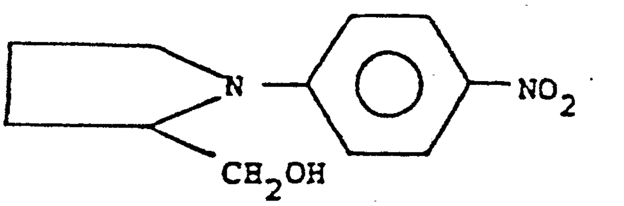

(I)

2. Paranitroaniline derivative corresponding to the general formula:

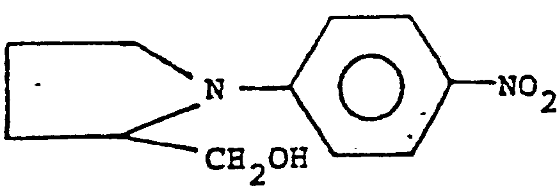

(I)

in which one or more of the hydrogen atoms are replaced by deuterium atoms.

3. Process for the preparation of a paranitroaniline derivative according to claim 1, characterized in that it comprises reacting 2-(hydroxymethyl)pyrrolidine having the formula II

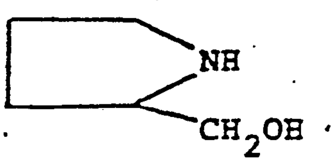

(II)

with a parahalogenonitrobenzene of the formula III

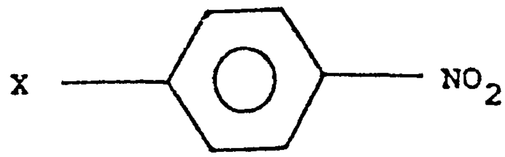

(III)

in which X represents a halogen atom.

4. Process according to claim 3, characterized in that X represents fluorine.

5. Process for the preparation of a paranitroaniline derivitive according to claim 2, characterized in that it consists in reacting 2-(hydroxymethyl)pyrrolidine of the formula II

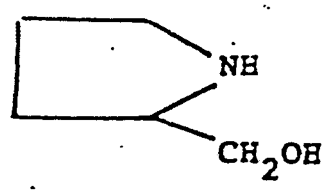

(II)

with parahalogenonitrobenzene of the formula III

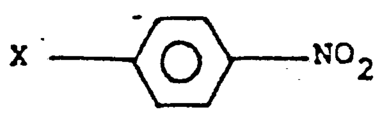

(III)

in which X represents a halogen atom and in deuterating the paranitroaniline derivätive thereby obtained.

6. Process according to any one of claims 3 to 5, characterized in that one or other of the two enantiomers S (+) or R(−) of 2-(hydroxymethyl)pyrrolidine are employed.

7. The use of optically pure paranitroaniline derivatives according to either of claims 1 and 2, in optical or electron-optical apparatus.